(19) Europäisches Patentamt
European Patent Office
Office européen des brevets

(11) EP 3 388 328 A1

# (12) EUROPEAN PATENT APPLICATION

published in accordance with Art. 153(4) EPC

(43) Date of publication:
**17.10.2018 Bulletin 2018/42**

(21) Application number: **16872720.4**

(22) Date of filing: **21.10.2016**

(51) Int Cl.:
***B63C 11/02*** *(2006.01)* ***A63B 29/00*** *(2006.01)*
***A63B 71/06*** *(2006.01)* ***A63C 11/00*** *(2006.01)*

(86) International application number:
**PCT/JP2016/081374**

(87) International publication number:
**WO 2017/098820 (15.06.2017 Gazette 2017/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **07.12.2015 US 201562263881 P**

(71) Applicant: **Sony Corporation**
**Tokyo 108-0075 (JP)**

(72) Inventors:
- **IKENOYA, Yuichiro**
  **Tokyo 108-0075 (JP)**
- **MIYAZAKI, Takuya**
  **Tokyo 108-0075 (JP)**
- **SUZUKI, Shunsuke**
  **Tokyo 108-0075 (JP)**

(74) Representative: **MFG Patentanwälte**
**Meyer-Wildhagen Meggle-Freund**
**Gerhard PartG mbB**
**Amalienstraße 62**
**80799 München (DE)**

## (54) INFORMATION PROCESSING DEVICE, INFORMATION PROCESSING METHOD, PROGRAM, AND INFORMATION PROCESSING TERMINAL

(57) [Object] To provide an information processing device, information presentation method, program, and information processing terminal that are capable of generating optimal behavior recommendation information in a specific environment in view of individual daily data.

[Solution] The information processing device includes a generation unit configured to generate behavior recommendation information to a user in a specific environment on a basis of log information related to the user in a daily environment.

FIG. 1

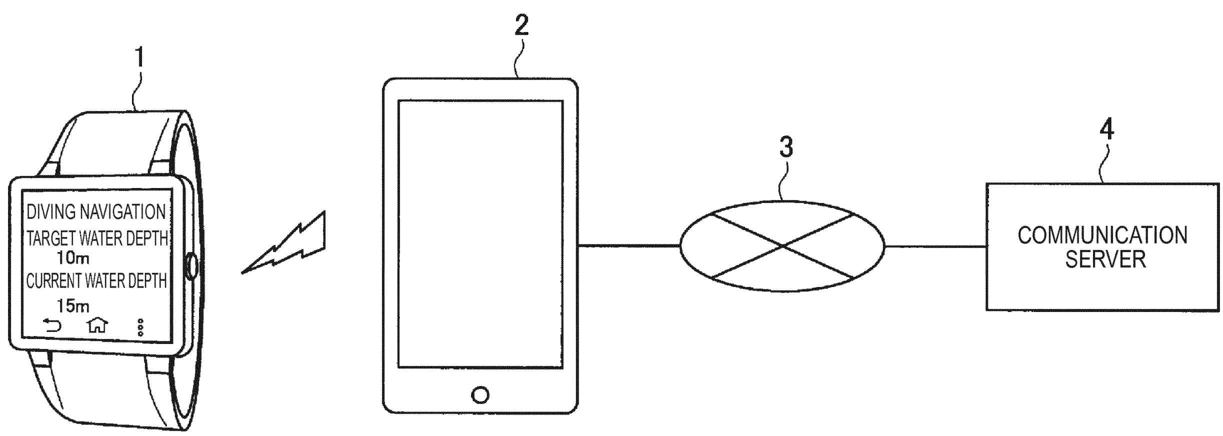


EP 3 388 328 A1

## Description

Technical Field

The present disclosure relates to information processing devices, information processing methods, programs, and information processing terminals.

Background Art

In general, in underwater diving, a maximum water depth and dive duration are decided by using a dive table or a dive computer to plan a dive.

In the underwater diving, a diver wears a diving watch on his/her wrist and dives into water. The diving watch detects information such as a water depth, diveable duration, and a water temperature by a sensor, and displays the detected information as information necessary for underwater diving, for example. In addition, it is possible to know an amount of air remaining in a diving cylinder by seeing a pressure gauge.

Note that, if pieces of necessary information are displayed in different display units installed at separate positions, it is inconvenient to quickly read the pieces of necessary information during underwater diving. Therefore, for example, Patent Literature 1 listed below proposes a device for displaying diver aid information in concentrated form to make a diver enjoy underwater diving more while securing safety of the diver by displaying pieces of information from a plurality of sensors in the concentrated form.

Citation List

Patent Literature

Patent Literature 1: JP H8-266685A

Disclosure of Invention

Technical Problem

Here, in dive plans proposed by conventional dive computers, individual personalities are not considered. Therefore, such dive plans are stereotypical. In addition, the dive computers are used only during underwater diving, and the dive computers are not used for acquiring and utilizing daily data.

Therefore, the present disclosure proposes an information processing device, information presentation method, program, and information processing terminal that are capable of generating optimal behavior recommendation information in a specific environment in view of individual daily data.

Solution to Problem

According to the present disclosure, there is provided an information processing device including a generation unit configured to generate behavior recommendation information to a user in a specific environment on a basis of log information related to the user in a daily environment.

According to the present disclosure, there is provided an information processing method including generating, by a processor, behavior recommendation information to a user in a specific environment on a basis of log information related to the user in a daily environment.

According to the present disclosure, there is provided a program causing a computer to function as a generation unit configured to generate behavior recommendation information to a user in a specific environment on a basis of log information related to the user in a daily environment.

According to the present disclosure, there is provided an information processing terminal to be worn by a user, the information processing terminal including a control unit configured to display behavior recommendation information to the user in a specific environment, the behavior recommendation information being generated on a basis of log information related to the user in a daily environment, and perform control such that a dive plan that is the behavior recommendation information is compared with a current dive situation of the user, and a warning is output in a case where the current dive situation deviates from the plan.

## Advantageous Effects of Invention

As described above, according to the present disclosure, it is possible to generate optimal behavior recommendation information in a specific environment in view of individual daily data.

Note that the effects described above are not necessarily limitative. With or in the place of the above effects, there may be achieved any one of the effects described in this specification or other effects that may be grasped from this specification.

## Brief Description of Drawings

[FIG. 1] FIG. 1 is a diagram illustrating an overview of an information processing system according to an embodiment of the present disclosure.

[FIG. 2] FIG. 2 is a block diagram illustrating an example of a configuration of a wearable device according to the embodiment.

[FIG. 3] FIG. 3 is a block diagram illustrating an example of a configuration of an information processing terminal according to the embodiment.

[FIG. 4] FIG. 4 is a sequence diagram illustrating a log information storage process in a daily life according to the embodiment.

[FIG. 5] FIG. 5 is a sequence diagram illustrating a plan generation process in a specific environment according to the embodiment.

[FIG. 6] FIG. 6 is a diagram illustrating display screen examples of the information processing terminal and the wearable device according to the embodiment.

[FIG. 7] FIG. 7 is a diagram illustrating display screen examples during and after underwater diving in an upskilling mode according to the embodiment.

[FIG. 8] FIG. 8 is a diagram illustrating an example of display screens in a diving workshop according to the embodiment.

[FIG. 9] FIG. 9 is a diagram illustrating an example of a mountaineering navigation display screen according to the embodiment.

[FIG. 10] FIG. 10 is a diagram illustrating an example of a snowboarding navigation display screen according to the embodiment.

[FIG. 11] FIG. 11 is a diagram illustrating an overview of a function enhancement housing case according to the embodiment.

[FIG. 12] FIG. 12 is a block diagram illustrating an example of a configuration of the function enhancement housing case according to the embodiment.

[FIG. 13] FIG. 13 is a sequence diagram illustrating an operation process of the information processing system according to the embodiment.

## Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Note that, the description is given in the following order.

1. Overview of information processing system according to embodiment of present disclosure
2. Configuration
2-1. Configuration of wearable device 1
2-2. Configuration of information processing terminal 2
3. Operation process
4. Plan generation example
4-1. Generation of dive plan in view of health condition
4-2. Generation of dive plan in view of beginner diver
4-3. Generation of plan for diving workshop
4-4. Usage example for mountaineering
4-5. Usage example for skiing or snowboarding

4-6. Generation of plan in view of situation of another user
5. Function enhancement housing case
6. Conclusion

<<1. Overview of information processing system according to embodiment of present disclosure>>

FIG. 1 is a diagram illustrating an overview of an information processing system according to an embodiment of the present disclosure. As illustrated in FIG. 1, the information processing system according to the embodiment includes a wearable device 1 and an information processing terminal 2. The wearable device 1 and the information processing terminal 2 may be connected in a wired/wireless manner, and may exchange data with each other. In addition, the information processing terminal 2 may connect with a communication server 4 such as a social networking service (SNS) via the Internet 3, and may exchange data.

(Background)

Here, as described above, in dive plans proposed by conventional dive computers, individual personalities are not considered. Therefore, such dive plans are stereotypical. In addition, the dive computers are used only during underwater diving, and the dive computers are not used for acquiring and utilizing daily data.

However, if making a plan for a sport such as underwater diving or snowboarding regardless of an individual health condition, problems may occur such as safety issues like an accident or injury, or a lack of satisfaction like a case where exercise is not enough.

In such a case, the information processing system according to the embodiment is capable of generating optimal behavior recommendation information in a specific environment such as underwater diving or snowboarding (sport played under water or above a ground) by using personal log information such as biological information, an amount of exercise in a daily environment, or the like.

Specifically, first, it is possible to detect biological information and an amount of daily exercise and record them as log information in the case where the user routinely wears the wearable device 1, for example. Such log information is transferred to the information processing terminal 2 and accumulated. The information processing terminal 2 generates an optimal plan for a specific environment by using the accumulated log information (hereinafter, also referred to as personal log information) and individual information (hereinafter, also referred to as personal information) such as age, weight, and height. The generated plan is transmitted from the information processing terminal 2 to the wearable device 1, and provided to the user in the specific environment.

In addition, the wearable device 1 worn by the user displays a navigation screen corresponding to the optimal plan in the specific environment such as underwater diving (under water). The user follows the navigation and enjoy underwater diving or the like with high safety and satisfactory.

In addition, the information processing terminal 2 is also capable of acquiring a situation of a member who accompanies the user from the communication server 4, and optimizing the plan in view of the situation of the member (personal information and personal log information).

In addition, the information processing terminal 2 is also capable of acquiring plans of other users from the communication server 4 and introducing another user having a similar plan.

The overview of the information processing system according to the embodiment of the present disclosure has been described. Next, respective structural elements of the information processing system according to the embodiment will be described.

<<2. Configuration>>

<2-1. Configuration of wearable device 1>

FIG. 2 is a block diagram illustrating an example of a configuration of the wearable device 1 according to the embodiment. As illustrated in FIG. 2, the wearable device 1 includes a control unit 10, a communication unit 11, an operation input unit 12, a sensor 13, an output unit 14, a log information storage unit 15, and a time measurement unit 16.

The control unit 10 functions as an arithmetic processing device and a control device, and controls the overall operation in the wearable device 1 in accordance with various programs. For example, the control unit 10 is implemented by a central processing unit (CPU), and an electronic circuit such as a microprocessor or the like. In addition, the control unit 10 may include read only memory (ROM) for storing programs, arithmetic parameters, and the like to be used, and random access memory (RAM) for temporarily storing parameters and the like that arbitrarily change.

In addition, the control unit 10 according to the embodiment stores various kinds of information detected by the sensor 13 in a daily life, in the log storage unit 15 as log information. In addition, in a specific environment, the control unit 10 displays a predetermined plan on the output unit 14, compares content of the plan with a current situation of a user on the basis of the information detected by the sensor 13, and outputs a warning from the output unit 14 in the case where the current situation deviates from the content of the plan.

The communication unit 11 is an interface (I/F) configured to connect with an external device in a wired/wireless manner and exchange data. For example, the communication unit 11 connects with the information processing terminal 2 via Bluetooth (registered trademark), Wi-Fi (registered trademark), a wired/wireless local area network (LAN), or the like, transmits log information to the information processing terminal 2, and receives a plan for a specific environment from the information processing terminal 2. In addition, the communication unit 11 is capable of communicating with a wearable device 1 of another user during underwater diving via ultrasonic communication or wired communication, and acquiring data indicating situations of pieces of equipment such as an amount of air remaining in a diving cylinder (for example, tank pressure) or the like of a user. The ultrasonic communication and wired communication are useful even in underwater situations.

The operation input unit 12 receives an operation instruction from a user, and outputs content of the operation to the control unit 10. The operation input unit 12 may be a touch sensor, a pressure sensor, or a proximity sensor, for example. Alternatively, the operation input unit 12 may be a physical structural element such as a button, a switch, or a lever.

The sensor 13 includes various kinds of sensors configured to detect situations of a user. Examples of the situations of a user include daily behavior recognition information, biological information (heartbeats, body temperature, sweating, blood pressure, sweating, pulse, breathing, blinking, eye movement, gaze duration, a pupil diameter size, blood pressure, brainwaves, body motion, posture, skin temperature, electric resistance of skin, micro-vibration (MV), myoelectric potential, SpO2 (blood oxygen saturation level)), feeling information, user attitude, positional information, surrounding environment information (illuminance, place, behavior history, surrounding circumstance, time, altitude, air temperature, wind direction, wind speed, etc.), and the like. To acquire such information, the various kinds of sensor includes a gyro sensor, an acceleration sensor, a geomagnetic sensor, a biosensor (heartbeats, body temperature, sweating, blood pressure, sweating, pulse, breathing, blood pressure, brainwaves, body motion, posture, skin temperature, electric resistance of skin, micro-vibration (MV), myoelectric potential, SpO2 (blood oxygen saturation level)), a positional information acquisition unit such as a global navigation satellite system (GNSS), a illuminance sensor, a barometric sensor, a camera, a temperature (air temperature) sensor, an altitude sensor, and the like, for example. The feeling information may be generated on the basis of the biological information and the behavior recognition information.

In addition, the wearable device 1 according to the embodiment further includes a water depth sensor and a water temperature sensor.

The output unit 14 has a function of outputting predetermined information under the control of the control unit 10. Specifically, for example, the output unit 14 may be implemented by a display unit configured to display a plan for a specific environment. The display unit may be a display device such as a liquid crystal display (LCD) device or an organic electro-luminescence (EL) display, for example. In addition, the output unit 14 may include a sound reproduction unit configured to reproduce a sound signal such as a voice or an alarm. In addition, the output unit 14 may have a function of haptically outputting a presentation through vibration, electrical stimulation, or the like.

The log information storage unit 15 is a storage unit configured to store various kinds of sensor information detected by the sensor 13 in a daily environment, as log information. In addition, the storage unit is implemented by read only memory (ROM) or random access memory (RAM). The ROM stores programs, arithmetic parameters, and the like that are used in processes performed by the control unit 10, and the RAM temporarily stores a parameters and the like that arbitrarily change.

In addition, the time measurement unit 16 measures time and outputs the measurement result to the control unit 10. For example, in the case of underwater diving, the time measurement unit 16 measures diving duration and outputs it to the control unit 10.

The detailed configuration of the wearable device 1 according to the embodiment has been described above. The wearable device 1 may have a watch-type shape as illustrated in FIG. 1, a glasses-type head-mounted display (HMD) shape, a wristband-type shape, or the like.

In addition, the wearable device 1 according to the embodiment is waterproof and pressure resistant in high extent because the wearable device 1 is assumed to be used under water.

<2-2. Configuration of information processing terminal 2>

Next, with reference to FIG. 3, a configuration of the information processing terminal 2 according to the embodiment will be described. FIG. 3 is a block diagram illustrating an example of the configuration of the information processing terminal 2 according to the embodiment.

As illustrated in FIG. 3, the information processing terminal 2 according to the embodiment includes a control unit 20, a communication unit 21, an operation input unit 22, a display unit 23, a personal information database (DB) 24, a personal log information DB 25, and an algorithm DB 26.

(Control unit 20)

The control unit 20 functions as an arithmetic processing device and a control device, and controls the overall operation in the wearable device 1 in accordance with various programs. For example, the control unit 20 is implemented by a central processing unit (CPU), and an electronic circuit such as a microprocessor or the like. In addition, the control unit 20 may include read only memory (ROM) for storing programs, arithmetic parameters, and the like to be used, and random access memory (RAM) for temporarily storing parameters and the like that arbitrarily change.

In addition, the control unit 20 according to the embodiment functions as a log storage control unit 201, an application decision unit 202, a plan generation unit 203, and a score calculation unit 204.

The log storage control unit 201 performs control such that the personal log information DB 25 stores log information received from the wearable device 1 via the communication unit 21.

The application decision unit 202 decides an application to be used. Such applications are prepared for respective specific environments, for example. The applications are assumed to include an underwater diving application, a mountaineering application, a snowboarding application, a bicycle application, and the like. The application decision unit 202 may decide an application on the basis of a selection made by a user, or the application decision unit 202 may automatically decide an application on the basis of real-time sensor information notified by the wearable device 1. For example, the application decision unit 202 is capable of selecting an underwater diving application in accordance with a result of detection performed by the water depth sensor.

The plan generation unit 203 generates behavior recommendation information (hereinafter, referred to as a plan) to a user in a specific environment on the basis of log information accumulated in the personal log information DB 25. More specifically, the plan generation unit 203 generates a plan for a specific environment in accordance with the application decided by the application decision unit 202. When the application is executed, a corresponding algorithm is extracted from the algorithm DB 26. For example, in the case where the underwater diving application is selected, the plan generation unit 203 executes an algorithm corresponding to the underwater diving application, and generates (calculates) a dive plan (such as adequate depth (maximum and average) and dive duration, for example) in accordance with log information (such as health condition) of the user.

The display unit 23 presents the plan generated by the plan generation unit 203 to the user. In the case where the user accepts it, the communication unit 21 transmits the plan to the wearable device 1. Next, the plan is presented through the wearable device 1 and navigation according to the plan starts. For example, when adequate dive duration and adequate maximum depth are displayed in diving navigation, it is possible for a diver to consciously manage his/her depth and dive in a more safe way.

Note that, details of plan generation according to the embodiment will be described in "4. Plan generation example".

The score calculation unit 204 compares the proposed and accepted plan with content of actual behavior of the user, and calculates a score for evaluating whether the user has behaved in accordance with the plan. The calculated score may be presented to the user via the display unit 23.

(Communication unit 21)

The communication unit 21 is an interface (I/F) configured to connect with an external device in a wired/wireless manner and exchange data. For example, the communication unit 21 connects with the wearable device 1 via Bluetooth (registered trademark), Wi-Fi (registered trademark), a wired/wireless local area network (LAN), or the like, receives log information from the wearable device 1, and transmits the plan for the specific environment to the wearable device 1. In addition, it is also possible for the communication unit 21 to connect with the Internet 3 and exchange data with a predetermined server (such as the communication sever 4) on the network.

(Operation input unit 22)

The operation input unit 22 detects operation input by the user, and outputs a detected input signal to the control unit 20. The operation input unit 22 may be implemented by a touchscreen, a switch, a button, or the like.

(Display unit 23)

The display unit 23 is a display device configured to output a plan generated by the plan generation unit 203, and various kinds of screens such as a health information input screen and the like. The display unit 23 may be a display device such as a liquid crystal display (LCD) device or an organic electro-luminescence (EL) display, for example.

(Personal information DB 24)

The personal information DB 24 is a storage unit configured to store personal information such as age, weight, height, and the like of a user.

(Personal log information DB 25)

The personal log information DB 25 is a storage unit configured to store log information (lifelog) such as an amount of exercise, health conditions (specifically, biological information), behavior recognition information, or the like of a user in his/her daily environment. In addition, the personal log information DB 25 is also capable of storing behavior content histories in a specific environment such as underwater diving information.

(Algorithm DB 26)

The algorithm DB 26 is a storage unit configured to store algorithms to be used when executing respective applications for specific environments.

The above-described storage unit for storing the personal information DB24, the personal log information DB 25, and the algorithm DB 26 is implemented by read only memory (ROM) and random access memory (RAM). The ROM stores programs, arithmetic parameters, and the like that are used in processes performed by the control unit 20, and the RAM temporarily stores a parameters and the like that arbitrarily change.

The configuration of the information processing terminal 2 according to the embodiment has been described above. Note that, the configuration of the information processing terminal 2 is not limited to the example illustrated in FIG. 3. For example, the information processing terminal 2 may further include a microphone and a speaker to input and output sound. In addition, the information processing terminal 2 may be implemented not only by the smartphone illustrated in FIG. 1, but also by a tablet terminal, a mobile phone terminal, a wearable terminal (such as an HMD, a glasses-type see-through wearable terminal (smartglasses), a smart band, a smartwatch, or a smart neck), a game console, a music player, or the like.

<2-4. Operation process>

Next, with reference to FIG. 4 and FIG. 5, operation processes of the information processing system according to the embodiment will be described.

<2-4-1. Log information storage process in daily life>

FIG. 4 is a sequence diagram illustrating a log information storage process in a daily life according to the embodiment.

First, as illustrated in FIG. 4, the wearable device 1 activates a health management application (Step S103), and starts connecting with the information processing terminal 2 (Step S106). For example, the connection with the information processing terminal 2 may be established via near-field communication such as Wi-Fi or Bluetooth.

Next, the wearable device 1 starts sensing biological information, an amount of exercise, or the like by using the sensor 13 (Step S109), and transmits various kinds of sensing data obtained through the sensing to the information processing terminal 2 (Step S112).

Next, the information processing terminal 2 stores the various kinds of received sensing data as log information (Step S115).

The above-described storage process in Step S109 to Step S115 is continuously repeated in a daily life. This enables to accumulate an amount of daily exercise and biological information of the user. Note that, it is also possible to temporarily accumulate log information to be transmitted to the information processing terminal 2 in the wearable device 1, and transmit the accumulated log information when communication with the information processing terminal 2 is established.

<2-4-2. Plan generation process in specific environment>

Next, with reference to FIG. 5, a process for generating a plan for a specific environment by using the accumulated log information will be described. FIG. 5 is a sequence diagram illustrating a plan generation process for a specific environment according to the embodiment.

First, as illustrated in FIG. 5, the information processing terminal 2 selects an application in accordance with a decision made by the application decision unit 202, and activates the application (Step S203). For example, the information processing terminal 2 activates an underwater diving application in accordance with an instruction from a user.

Next, the plan generation unit 203 acquires health information accumulated in the personal log information DB 25 (here, as an example, health information that is biological information included in log information is used) (Step S206).

Next, the information processing terminal 2 acquires a current health condition of the user (Step S206). For example, the information processing terminal 2 may display a screen through which the user inputs his/her current health condition on the display unit 23 and acquire the health information of the user.

Next, the plan generation unit 203 generates a dive plan on the basis of the health information (Step S209). For example, the plan generation unit 203 calculates a maximum adequate depth and adequate dive duration as the dive plan, on the basis of an average blood pressure value and sleep duration of the user, information indicating whether the user has drunk alcohol yesterday, or the like.

Next, the information processing terminal 2 provides the generated dive plan to the user, and waits for acceptance for usage (Step S212).

In the case where the acceptance is not received (NO is Step S212), the information processing terminal 2 generates another dive plan (Step S209).

On the other hand, in the case where the acceptance is received (YES in Step S212), the information processing terminal 2 transmits plan information to the wearable device 1 (Step S215).

Next, the wearable device 1 conducts diving navigation on the basis of the plan information (Step S218). Specifically, in general, the wearable device 1 activates the health management application and acquires an amount of exercise and health conditions of the user in his/her daily life. In the case where plan information for a specific environment is transmitted from the information processing terminal 2, the wearable device 1 activates a corresponding application and displays a navigation screen based on the plan information.

Here, with reference to FIG. 6, display screen examples of the information processing terminal 2 and the wearable device 1 according to the embodiment will be described. Here, display screen examples displayed in the case where the underwater diving application is activated are illustrated, for example.

As illustrated in the left side of FIG. 6, an input screen 130 through which the user inputs his/her current health condition into the information processing terminal 2 is displayed before underwater diving. Through the input screen 130, it is possible for the user to input his/her subjective health condition (select one of face icons indicating a very good condition, good condition, normal condition, and bad condition), input yesterday's sleeping duration, and input whether he/she has drunk alcohol yesterday, for example. Note that, such input content in the input screen 130 is a mere example. The embodiment is not limited thereto.

Next, as illustrated in the middle of FIG. 6, the wearable device 1 displays a navigation screen 140 during underwater diving on the basis of the generated plan information. For example, the navigation screen 140 displays a target water depth and a current water depth. This enables the user to check the target water depth even during underwater diving. Accordingly, it is possible for the diver to instantly understand his/her optimal depth, and stop diving into a depth that exceeds his/her ability. Note that, in the example illustrated in FIG. 6, a main body part of the wearable device 1 is covered by a waterproof and pressure resistant case 18, for example.

Next, after the dive, the wearable device 1 displays an advice screen 141 that displays time before a next dive and the like as illustrated in the right side of FIG. 6. Specifically, by using the underwater diving application activated in the information processing terminal 2, it is possible to calculate time before the next dive (necessary recess time) and a target water depth or the like in the next dive, and display them on the wearable device 1 as advice for the user.

The operation processes of the information processing system according to the embodiment have been described above in detail.

## <<4. Plan generation example>>

Next, detailed examples of generation of a plan for a specific environment according to the embodiment will be described.

### <4-1. Generation of dive plan in view of health condition>

First, in an example of generating a dive plan in view of a health condition, it is possible to calculate an optimal maximum adequate depth in view of blood pressure of a user, for example. This is highly convenient for a diver who has high blood pressure.

Specifically, the plan generation unit 203 calculates latest average values of systolic blood pressure and diastolic blood pressure from blood pressure data that are measured in a daily life of a user and accumulated in the personal log information DB 25. In addition, as a current health condition of the user, the plan generation unit 203 acquires blood pressure data of the user measured on the day. In addition, the plan generation unit 203 acquires diving certification information of the user from the personal information DB 24 (the user has input his/her certification information in advance), and acquires a maximum diveable depth corresponding to his/her certification level.

Next, the plan generation unit 203 is capable of calculating the maximum adequate depth from the following equation 1, while using the average values of systolic blood pressure and diastolic blood pressure of the user acquired in the above-described way, the systolic blood pressure and diastolic blood pressure measured on the day, and the maximum diveable depth corresponding to the certification level.

[Math. 1]

Maximum adequate depth (in view of blood pressure)

$$\text{Maximum adequate depth (in view of blood pressure)} = \frac{\text{Systolic blood pressure (average value)}}{\text{Measured systolic blood pressure}} \times \frac{\text{Diastolic blood pressure (average value)}}{\text{Measured diastolic blood pressure}} \times \text{Maximum diveable depth}$$

・・・Equation 1

In addition, it is possible for the plan generation unit 203 to adjust the maximum diveable depth information in accordance with marine information (such as weather, air temperature, water temperature, wind speed, wind direction, ocean current, and landform) of a dive site on the day in the case where the marine information of the dive site can be acquired. The dive site may be manually input by the user, or may be recognized on the basis of current position information acquired from the positional information acquisition unit installed in the wearable device 1 or the information processing terminal 2.

In addition, the plan generation unit 203 is capable of modifying the maximum adequate depth in accordance with age of the user. For example, in the case of underwater diving, a medical examination by a doctor is recommended for divers 40 years of age or older before underwater diving. Therefore, it is possible to calculate a maximum adequate depth for a user 40 years of age or older by using the following equation 2.

[Math. 2]

$$\text{Maximum adequate depth(in view of age)} = \text{Maximum diveable depth} \times \frac{40}{\text{One's age}}$$

・・・Equation 2

In addition, in the case of a diver who is 40 years of age or older and has high blood pressure, the plan generation unit 203 substitutes the maximum adequate depth calculated from the above-listed equation 1 (in view of blood pressure) for the "maximum diveable depth" in the above-listed equation 2, to further modify the maximum adequate depth.

In addition, the plan generation unit 203 is also capable of further modifying the maximum adequate depth in accordance with a simple self-diagnosis by a user himself/herself (input his/her health condition). For example, the plan generation unit 203 is capable of prompting the user to input details of yesterday's drinking via an input screen of the information processing terminal 2, and deciding his/her maximum adequate depth and dive duration in accordance with the details of his/her drinking by using the following equation 3 and table 1. The details of drinking are input by the user on a four-point scale, for example. The plan generation unit 203 calculates the maximum adequate depth (in view of the self-diagnosis) by subtracting a difference corresponding to the details of drinking from the maximum diveable depth of the user or the maximum adequate depth calculated from the above-listed equation 1 or 2.

[Math. 3]

$$\text{Maximum adequate depth (in view of self-diagnosis)} = \text{Maximum diveable depth (or maximum adequate depth)} - \text{Maximum depth difference}$$

・・・Equation 3

[Table 1]

| | Amount of drinking | | | |
|---|---|---|---|---|
| Question content | Very large | Large | Small | Did not drink |
| Maximum depth difference | -4 m | -3 m | -2 m | 0 m |

Accordingly, the wearable device 1 displays the maximum adequate depth before and during underwater diving in order to prevent a dive depth of the user from exceeding the maximum adequate depth calculated as described above. In the case where the dive depth of the user exceeds the maximum adequate depth during underwater diving, the wearable device 1 outputs a warning from the output unit 14. Ways of warning include output of warning display and output of a warning alarm, for example.

<4-2. Generation of dive plan in view of beginner diver>

Next, generation of a dive plan for a beginner diver (in the upskilling mode) will be described. For example, the beginner diver is defined here as a diver having 20 lifetime dives or less. The plan generation unit 203 is capable of acquiring dive history information of the user from the personal log information DB 25, and recognizing the number of lifetime dives, dive sites where the user has dived before, date and time of the last dive, members who have dived together, or the like. Next, the plan generation unit 203 determines that the user is the beginner diver in the case where the user has 20 lifetime dives or less. Subsequently, the plan generation unit 203 calculates a maximum adequate depth and dive duration corresponding to the number of lifetime dives, as illustrated in a table 2 listed below.

[Table 2]

| Number of lifetime dives (excluding workshop) | 0 to 5 dives | 6 to 10 dives | 11 to 15 dives | 16 to 20 dives |
|---|---|---|---|---|
| Maximum adequate depth | 14 m | 15 m | 16 m | 17 m |
| Maximum dive duration | 34 minutes | 36 minutes | 38 minutes | 40 minutes |

Note that, in accordance with the number of lifetime dives, the plan generation unit 203 is also capable of modifying the maximum adequate depth and dive duration that have been determined from a health condition of the user based on the log information.

In addition, the wearable device 1 is capable of displaying the maximum adequate depth and dive duration based on the plan during underwater diving, displaying a dive depth and dive duration of the user in real time, and supporting the user such that the user can maintain his/her target water depth even in if the user is a beginner diver.

Next, after the underwater diving, the score calculation unit 204 is capable of quantifying content of the latest dive on the basis of a deviation rate of the user from his/her maximum adequate depth and dive duration. Here, the following equation 4 is an example of the score calculation.

[Math. 4]

$$\text{Score} = \frac{\text{Duration of deviation from maximum adequate depth}}{\text{Entire dive duration}} \times 100$$

$\cdot\cdot\cdot$ Equation 4

As described above, the user is provided with the quantified content of the latest dive after the underwater diving. This enables the user to easily understand his/her level. The calculated score is stored in the personal log information DB 25 in association with dive information. In addition, the plan generation unit 203 calculates an appropriate maximum adequate depth and dive duration for a next dive in accordance with the score, and presents them to the user in addition to the score. This enables to help improvement in the user's skill.

Here, FIG. 7 illustrates an example of display screens during and after underwater diving in the upskilling mode. As illustrated in the left side of FIG. 7, the wearable device 1 displays a navigation screen 142 during underwater diving.

The navigation screen 142 displays a target water depth and a current water depth. The navigation screen 142 displays how long the diver should stay at a current water depth, and displays advice or the like to achieve it. If the beginner diver follows the instruction displayed on the navigation screen 142 while underwater diving, his/her skill will be improved.

In addition, as illustrated in the right side of FIG. 7, the information processing terminal 2 displays a result screen 131 of content of the latest dive after the underwater diving. The result screen 131 displays the latest target water depth, an average water depth of the user, a score, and advice about this result. Accordingly, the user is expected to achieve effects such as improvement in buoyancy control, improvement in his/her skill while enjoying underwater diving as though it were a game thanks to the score display, or improvement in his/her skill without being taught by an advanced diver.

Note that, the plan generation unit 203 is also capable of adjusting the maximum adequate depth and maximum dive duration in accordance with dive sites. For example, it is possible to recognize dive sites where the user has dived before, date and time of past dives, dive depths, dive duration, and score information (to be described later) from the dive history information of the user. In the case of a dive site where the user has dived before, the maximum adequate depth and dive duration may be adjusted as illustrated in a table 3 listed below. This enables to evaluate individuals by using adequate scores corresponding to individual levels. In addition, it is possible to feed back the adequate scores to users.

[Table 3]

| Number of dives | 1 to 5 dives | 6 to 10 dives | 11 to 15 dives | 16 to 20 dives |
|---|---|---|---|---|
| Maximum adequate depth | +1 m | +2 m | +3 m | +4 m |
| Dive duration | +5 minutes | +10 minutes | +15 minutes | +20 minutes |

In addition, even if the user is a beginner diver, the plan generation unit 203 according to the embodiment is capable of generating a plan for the situation where a veteran diver accompanies the user in the case where it is recognized from the accompanying member information that the veteran diver accompanies the user. For example, the veteran diver is defined here as a diver having 100 lifetime dives or more and having 50 self-reliant dives or more.

Here, a table 4 listed below illustrates correction amounts of a maximum adequate depth and dive duration in the case where a veteran diver accompanies the user.

[Table 4]

| | |
|---|---|
| Maximum adequate depth | +5 m |
| Dive duration | +10 minutes |

In the case where a veteran diver accompanies a beginner diver, the plan generation unit 203 uses the correction amounts illustrated in the table 4 and modifies the maximum adequate depth and dive duration that have been calculated on the basis of the number of lifetime dives of the beginner diver as described above. However, the plan generation unit 203 modifies the maximum adequate depth and dive duration such that the maximum adequate depth does not exceed a maximum diveable depth corresponding to a certification level of the beginner diver.

<4-3. Generation of plan for diving workshop>

In addition, the plan generation unit 203 is capable of generating a dive plan in which an instructor holds a workshop for trainees in the sea.

In the underwater diving workshop, the trainees first studies on land, memorizes content, and then practices in the sea. Therefore, the instructor and trainees select ranks and content of the workshop via the information processing terminals 2 in advance. The diving application activated by the information processing terminal 2 transmits the plan information generated on the basis of content of the selected workshop to the wearable device 1, and presents the content of the workshop during underwater diving. In addition, display of tips for improving his/her skill helps a user who is not good at practicing to overcome his/her weakness.

Here, examples of the content of the workshop are listed below.

Workshop on hand signals: to learn about hand signals used under water.

Mask clearing: to learn how to clear water from a mask in case water gets into the mask.

Octo sharing: to learn what to do in case your buddy is out of air.

In addition, here, FIG. 8 illustrates an example of display screens in a diving workshop. When content of a workshop (here, "hand signals", for example) is selected via the information processing terminal 2 before underwater diving as illustrated in the left side of FIG. 8, the wearable device 1 displays the content of the workshop during underwater diving as illustrated in the right side of FIG. 8. Therefore, it is possible for a trainee to practice the content of the workshop while reviewing the content of the workshop.

As described above, in the "workshop on hand signals", the wearable device 1 displays how to make signals and meanings of the signals to make hand signals under water, and a trainee actually makes the hand signals while seeing the screen. In addition, the wearable device 1 displays only meanings of the hand signals after a predetermined period of time elapses in order to check whether the trainee memorizes the hand signal.

In addition, in the "workshop on mask clearing", the wearable device 1 displays a workflow to clear water from a mask in case water gets into the mask under water. The user practices the mask clearing while seeing the screen and checking the workflow. In the case where the user fails at the mask clearing, the wearable device 1 displays the workflow of mask clearing and tips for mask clearing again, and the user repeatedly practice it multiple times.

In addition, in the "workshop on octo sharing", two people shares one diving cylinder by using a secondary demand valve in case your buddy is low on air. In this case, the wearable device 1 displays a hose to be passed to the buddy, and a trainee follows the instruction and passes the hose to the buddy. In addition, it is difficult to keep his/her depth while sharing the octo. Therefore, the wearable device 1 outputs a warning in the case where the trainee makes a rapid ascent.

As described above, trainees are capable of reviewing the content of the workshop under water. This makes the trainees to join the workshop with easy minds. In addition, the instructor has to train a plurality of trainees simultaneously. However, each of the trainees reviews the content of the workshop by using a wearable device 1 worn by himself/herself, and the wearable device 1 helps improvement in skills. Therefore, the instructor is capable of paying more attention to safety.

In addition, the plan generation unit 203 is also capable of generating an optimal plan for workshop by calculating maximum adequate depths and dive duration that are suitable to improve skills of respective trainees in accordance with age, sex, log information, and skill acquisition information of the trainees, and points in the workshop.

<2-4-4. Usage example for mountaineering>

It is possible to use the wearable device 1 according to the embodiment not only under water but also above the ground. Examples thereof include usage in mountaineering.

(Generation of adequate mountaineering plan)

In the case where the specific environment indicates mountaineering, the plan generation unit 203 generates a mountaineering plan including an optimal altitude, walking speed, and the like for mountaineering on that day, on the basis of heartbeat information, walking speed information, or the like of the user in his/her daily environment. Subsequently, the plan generation unit 203 presents the generated mountaineering plan. In addition, during mountaineering, the wearable device 1 can present positional information and altitude information based on the GNSS, and the optimal altitude, walking speed, and the like based on the plan.

For example, the optimal altitude and walking speed can be calculated from the following equations 5 and 6, respectively [Math. 5]

$$\text{Optimal walking speed} = \text{Daily walking speed} \times \frac{\text{Average daily pulse}}{\text{Current pulse}}$$

$$\cdots \text{Equation 5}$$

[Math. 6]

$$\text{Optimal mountaineering altitude} = \text{Altitude of starting point} + ((\text{Altitude of mountaintop} - \text{Altitude of starting point}) \times \frac{\text{Average daily pulse}}{\text{Current pulse}})$$

・・・Equation 6

(Change in mountaineering plan due to weather)

In the case where weather is getting worse during mountaineering, the plan generation unit 203 is capable of recognizing the situation from barometric pressure information, changing the plan drastically, and proposing the plan change to a user in advance via the information processing terminal 2 or the wearable device 1. For example, in the case where the weather is predicted to get worse before mountaineering finishes, the plan generation unit 203 changes walking time and mountaineering altitude such that the user can go back to a starting point before the weather gets worse.

Here, FIG. 9 illustrates an example of a mountaineering navigation display screen according to the embodiment. As illustrated in FIG. 9, the wearable device 1 displays a mountaineering navigation screen 145 in which mountaineering duration, weather situation, and advice about mountaineering (such as "please go back within 1 hour") are displayed. In addition, it is also possible to display comparison between a target walking speed and a current walking speed, display a current position on a map, or display comparison between an optimal mountaineering altitude and a current altitude.

(Change in plan in accordance with health condition)

In addition, even during mountaineering, the plan generation unit 203 is capable of detecting heartbeats and wearing information from the wearable device 1, monitoring change in a physical condition, and changing the plan into an optimal plan that suits a current condition such as changing the speed into a speed corresponding to the physical condition. By always monitoring a pulse, it is possible to continuously perform calculation using the above-listed equations 5 and 6, and chancing the walking speed and the mountaineering altitude in accordance with a current situation (as the time elapses). In addition, in the case where extreme sweating is detected for example, it is possible to display a warning or output an alarm sound from the wearable device 1.

<2-4-5. Usage example for skiing or snowboarding>

In addition, it is possible to use the wearable device 1 according to the embodiment in the case of playing a winter sport such as skiing or snowboarding. The plan generation unit 203 is capable of selecting an appropriate course in the skiing and snowboarding on the basis of log information of a user, for example.

For example, the plan generation unit 203 displays a current sliding speed and a map of a ski resort on the basis of altitude information and a current position information according to the GNSS or the like, selects a course suitable to the user on the basis of past skiing/snowboarding histories and skill information of the user, and presents the selected course.

In addition, it is also possible for the plan generation unit 203 to display advanced courses or one of the advanced courses that the user has never tried, for the user who has the past skiing/snowboarding history and who is highly skilled. In addition, for a beginner, the plan generation unit 203 may present a low-pitched course that is not crowded in view of current congestion situations of courses.

In addition, in the case of selecting an appropriate course, the plan generation unit 203 is capable of deciding an appropriate sliding speed, sliding duration, the number of slides, inclination angles, difficulty levels of courses, or the like in view of amounts of daily exercise and health information of the user, and generating a plan indicating an appropriate ski resort or course.

Here, FIG. 10 illustrates an example of a snowboarding navigation display screen. As illustrated in FIG. 10, the wearable device 1 displays a snowboarding navigation screen 146 in which sliding duration, target duration, and a recommended course are displayed, for example. In addition, it is also possible to display comparison between a target sliding speed and a current sliding speed, weather, tricks to be challenged, or the like.

<2-4-6. Generation of plan in view of situation of another user>

Next, details of plan generation in view of a situation of another user will be described. The existing dive computers do not consider situations of divers who accompany a user. However, according to the embodiment, it is possible to make a determination in a comprehensive manner in view of situations of other members, generates an optimal dive plan for a whole team, and present the generated dive plan to the whole team in the case where there are a plurality of members.

(Use in underwater diving)

For example, information obtained through the above-described diving plan generation is transmitted to the communication server 4 or one of the information processing terminals 2, and the information is analyzed. For example, the plan generation unit 203 of one of the information processing terminal 2 analyzes log information, skills, current health condition information, or the like of the members, generates an optimal plan for a team, and transmits the generated plan to the other information processing terminals 2. It is also possible for the communication server 4 to perform such a process, and transmit a result of the process to the information processing terminals 2 of the respective members.

For example, in the case where the team includes less-experienced beginner divers, the plan generation unit 203 adjusts maximum adequate depths of other accompanying divers in accordance with a maximum adequate depth of the most inexperienced diver. More specifically, for example, in the case where a beginner diver A has 20 lifetime dives or less and the other accompanying divers B and C are general divers (having 20 lifetime dives or more), maximum adequate depths of the respective divers are set to depths indicated in the table 2 listed above.

In addition, in the case where the less-experienced beginner diver has a bad health condition, the plan generation unit 203 uses an equation 7 and a table 5 listed below to calculate a maximum adequate depth of the beginner diver. Note that, the maximum diveable depth is calculated by using the table 2 listed above. The plan generation unit 203 shares the calculated maximum adequate depth with the accompanying general divers, and changes the maximum adequate depth of the team.

[Math. 7]

$$\text{Maximum adequate depth (in view of health condition)} = \text{Maximum diveable depth} - \text{Maximum depth difference}$$

$$\cdots \text{Equation 7}$$

[Table 5]

| | Health condition | | | |
|---|---|---|---|---|
| Question content | Bad | Bit bad | Not so bad | Good |
| Maximum depth difference | -4 m | -3 m | -2 m | 0 m |

Alternatively, in the case where there is a less-experienced beginner diver but he/she has dived this dive site before, the plan generation unit 203 corrects the maximum adequate depth in accordance with the table 3 listed above. In addition, with such a correction, the maximum adequate depths of the accompanying general divers are also changed.

In addition, in the case of generating a plan for a workshop, it is possible to change information of instructions to be issued to individuals in accordance with the judgment of an instructor and skill proficiency levels, and prevent widening of gaps in skills between trainees.

For example, in the case where the trainees have different amounts of air from each other as the workshop goes on, the wearable devices 1 displays a way of breathing under water at predetermined intervals for users having decreasing amounts of air to warn the users, although the wearable devices 1 normally do not display the way of breathing.

In addition, the wearable devices 1 are capable of displaying hand signals that are frequently used in respective depths to prompt trainees to use them. For example, in general, trainees make an OK sign immediately after water entry. However, in the case where one of the trainees has often forgotten to make this hand signal, the instructor configures the setting for the wearable device 1 of this trainee in advance such that the OK sign is displayed immediately after water entry. This enables the trainee to notice by himself/herself without an individual instruction from the instructor.

In addition, by using the information processing system according to the embodiment, it is possible to introduce a third person having a similar dive plan to a user with reference to underwater diving history information of respective users and social networking sites (open underwater diving history information). For example, it is possible to extract users whose top 5 dive sites where they have dived often are matched with each other, and introduce them to each other. Alternatively, it is possible to extract users having similar dive duration, similar maximum adequate depths, and similar ranks (score, diving certification type, or the like) and introduce them to each other.

(Use in mountaineering)

In addition, the plan generation unit 203 is capable of chancing a mountaineering course or plan in accordance with health conditions of members in a mountaineering team.

For example, communication is established in advance between the wearable devices 1 and the information processing terminals 2 in the team, and pulse information and sweating information are being monitored in the team. This enables to optimize the mountaineering course and plan for the team such that the mountaineering course and plan becomes suitable to a person having a worst health condition in the team.

For example, in the case where a mountaineer A has a bad health condition, the plan generation unit 203 calculates his/her walking speed and adequate altitude by using the equations 5 and 6 listed above, and changes mountaineering plans for the other mountaineers B and C in the same team as the mountaineer A, on the basis of a result of the calculation.

In addition, the information processing terminals 2 are capable of continuously monitoring current position information of team members by using the GNSS or the like. In the case where the members get apart from each other, the wearable devices 1 issue warnings to all the members to prevent them from getting lost. In addition, for example, in the case where the mountaineer A is away from a member of the team by 20 meters or more in radius, the wearable device 1 is capable not only of warning the mountaineer A, but also of warning all the members in the team.

In addition, it is also possible for the information processing system according to the embodiment to share not only information of the team but also information of mountaineering routes of other teams mountaineering in the same place, and change the plan. For example, in the case where a mountaineering team A and a mountaineering team B are climbing the same mountain and the team B gets ahead than the team A and transmits rockfall information or rapid worsening weather information to the communication server 4 via the wearable device 1 or the information processing terminal 2, the communication server 4 notifies a wearable device 1 and an information processing terminal 2 of the subsequent team A of the rockfall information or the like. Accordingly, it is possible for the plan generation unit 203 to instantaneously acquire latest land shapes and weather information, and change the mountaineering course on the basis of the acquired information.

(Use in skiing or snowboarding)

In addition, when the information processing system according to the embodiment is used in skiing or snowboarding, it is possible to share time information or the like with other users.

For example, the information processing terminal 2 is capable of acquiring positional information of respective nearby users who are skiing or snowboarding with the wearable devices 1 worn by themselves in the similar way, from the communication server 4, and acquiring fastest time information of the nearby users.

In addition, the information processing terminals 2 and the communication server 4 are capable of ranking levels of the respective users on the basis of such fastest time or the number of slides, calculating ranks of the users in real time, transmitting results of the calculation to the wearable devices 1 via the network, and causing the wearable devices 1 to display the transmitted results. By showing clear goals such as the ranks or order, it is possible to help improvement in the user's skill.

In addition, it is also possible to share situations of courses via the communication server 4. It is possible for other users to select courses in accordance with snow conditions when the respective users provides information regarding the snow conditions of the courses via the wearable devices 1 or the information processing terminals 2. In addition, when a user inputs his/her preferable snow condition in advance, it is possible for the system to recognize latest snow conditions and propose a recommended course to the user.

<<5. Function enhancement housing case>>

Next, a housing case that is to be used for the wearable device 1 in a specific environment such as underwater usage will be described.

The purpose of conventional housing cases is merely to protect a target device (wearable device such as smartwatch) from water.

However, a housing case according to the embodiment of the present disclosure is capable of enhancing functions of the target device.

Therefore, even when the target device does not have dedicated hardware, in other words, even when the target device is a general-purpose device, for example, it is possible to use the general-purpose device as a dedicated device by putting the general-purpose device into the housing case with a dedicated structure according to the embodiment.

FIG. 11 illustrates an overview of the function enhancement housing case according to the embodiment. As illustrated in FIG. 11, for example, functions of a general-purpose wearable device 6 is enhanced by putting the wearable device 6 into a housing case 5 having an opening-closing mechanism 55.

Specifically, communication is established between the housing case 5 and the wearable device 6 in a wired/wireless manner, and data detected by a sensor installed in the housing case 5 or data subjected to a predetermined process is transmitted to the wearable device 6. This enables the general-purpose device to store log information in a specific environment.

For example, to store underwater diving logs, a dedicated device (dive computer) having a water depth sensor, water temperature sensor, and the like with a highly-waterproof and pressure-resisting structure is necessary. However, according to the embodiment of the present disclosure, the water depth sensor and the water temperature sensor are installed in the housing case 5, and the housing case 5 has the highly-waterproof and pressure-resisting structure. This enables to use a general-purpose device as a substitute for a dive computer by inputting the general-purpose device (wearable device 6) into the housing case 5 and establishing communication between them.

In this case, a dedicated underwater diving application is installed in advance in the wearable device 6. Therefore, it is possible to store sensing data (water temperature and water depth) transmitted from the housing case 5 and perform a process of displaying the sensing data in real time.

Note that, the device to be put in the housing case 5 may be the above-described general-purpose device (wearable device 6), or may be the wearable device 1 according to the above-described embodiment. In addition, the wearable device 1 to be put into the housing case 5 does not have to include the sensors dedicated to a specific environment (such as water depth sensor and water temperature sensor) from among the structural elements described with reference to FIG. 2.

<5-1. Configuration>

Next, with reference to FIG. 12, a configuration of the housing case 5 according to the embodiment will be described. As illustrated in FIG. 12, the housing case 5 includes a measurement sensor 50, an operation input unit 51, an amplifier/ADC 52, a data/control bus 53, ROM 54, RAM 55, a CPU 56, a built-in battery 57, an external electric power input unit 58, and a communication unit 59.

The measurement sensor 50 is a water temperature measurement sensor, a water depth measurement sensor, and the like, for example. Here, the wearable device 6 that is the general-purpose device is used as a substitute for a dive computer, for example. Therefore, the examples of the measurement sensor 50 include sensors configured to measure various kinds of data that is necessary for underwater diving. However, the embodiment is not limited thereto.

The operation input unit 51 is an input unit configured to receive operation input by a user (control instruction). The operation input unit 51 may be a physical structural element such as a button, a switch, or a lever. This enables to power on/off the housing case 5, for example.

The amplifier/ADC (analog to digital converter) 52 digitizes data input from the measurement sensor 50 or the operation input unit 51, and output the digitized data to the data/control bus 53.

The data/control bus 53 is a transmission path of digital signals (data) in the housing case 5.

The ROM 54 stores programs, arithmetic parameters, and the like used by the CPU 56 or other structural elements. The RAM 55 transiently stores parameters or the like that change as appropriate. The CPU 56 is an example of an electronic circuit that functions as an arithmetic processing device and a control device to control all of operation performed in the housing case 5 in accordance with various kinds of programs.

The built-in battery 57 is a power source unit of the housing case 5. The built-in battery 57 is capable of receiving electric power supplied from the external electric power input unit 58.

The communication unit 59 connects with external devices in a wired/wireless manner and transmits/receives data. The communication unit 59 according to the embodiment connects with the wearable device 6 in the housing case 5 and transmits/receives data.

<5-2. Operation process>

Next, with reference to FIG. 13, an operation process according to the embodiment will be described. FIG. 13 is a sequence diagram illustrating an operation process in the information processing system according to the embodiment.

First, as illustrated in FIG. 13, the wearable device 6 installs a dedicated application (such as underwater diving application) (Step S303).

Next, when the housing case 5 detects that the wearable device 6 is stored (Step S306), the housing case 5 starts communication connection with the wearable device 6 (Step S309).

Next, the wearable device 6 also starts communication connection with the housing case 5 (Step S312), and starts an underwater diving mode (Step S315). In other words, a user first puts the wearable device 6 into the housing case 5 before underwater diving to take a measure to protect the wearable device 6 from water (seawater). The wearable device 6 in the housing case 5 receives a signal from the communication unit 59 of the housing case 5 via the dedicated application, and enters an underwater diving mode.

Next, the housing case 5 measures a water temperature and a water depth (Step S318), and continuously transmits measurement values of the measured water temperature and water depth to the wearable device 6 (Step S321).

Next, the wearable device 6 starts measurement of underwater diving time (dive duration) (Step S324) when start of underwater diving is detected on the basis of the received water depth value.

Next, the wearable device 6 displays the received measurement values of water temperature and water depth in real time (Step S327), calculates an amount of residual nitrogen on the basis of the received measurement values, and displays the calculated amount (Step S330).

Next, the wearable device 6 stores the received measurement values as log data (Step S333).

As described above, during underwater diving, the wearable device 6 is capable of receiving values measured by the measurement sensor 50 installed in the housing case 5 and thereby operating as a dive computer.

Next, in the case where the wearable device 6 detects end of the underwater diving on the basis of the water depth value (YES in Step S336), the wearable device 6 ends the measurement of the underwater diving time (Step S339).

Next, when the user takes the wearable device 6 from the housing case 5 and the housing case 5 detects that the wearable device 6 is removed (Step S342), the housing case 5 disconnects the communication with the wearable device 6 (Step S345).

Accordingly, the wearable device 6 also disconnects the communication with the housing case 5 (Step S348).

Next, the wearable device 6 transmits the log data to a predetermined external device such as the information processing terminal 2 (Step S351).

As described above, the wearable device 6 stores the logs during underwater diving. After the underwater diving, it is possible for external device such as the information processing terminal 2 to take out the data.

The operation processes of the information processing system according to the embodiment have been described above.

As described above, according to the embodiment, it is possible to enhance the functions of the wearable device 6 such as measurement of a water temperature and a water depth by simply putting the wearable device 6 into the waterproof housing case 5. Since the housing case 5 (measurement device) and the wearable device 6 (device to be housed) are independent from each other, any device can be used as the dedicated device (such as a dive computer) as long as the device has a communication unit compliant with a standard same as the communication unit of the housing case 5.

In addition, during underwater diving, the functions of the wearable device 6 (device to be housed) depends on installed applications. Therefore, it is possible to use additional functions under water by enhancing the applications. For example, it is possible to use the wearable device 6 as an undersea logger by using a MAP function. In addition, when using a large housing case 5, it is possible to apply the present technology not only to the small wearable device 6 but also to an information processing terminal such as a smartphone to a tablet terminal. In addition, a smartphone in the housing case 5 can be used as an underwater camera when a part of the shape of the housing case 5 is changed to use a camera of the smartphone in the housing case 5.

## <<5. Conclusion>>

As described above, by using the information processing system according to the embodiment of the present disclosure, it is possible to generate optimal behavior recommendation information for a specific environment in view of individual daily data.

For example, since individual health information is considered, a satisfaction level and a safety level of underwater diving are improved.

In addition, it is also possible to pair members having similar health conditions in a team. This can improve the satisfaction level of underwater diving.

In addition, in the case where a plurality of members have their own wearable devices 1, it is also possible to acquire log data that is similar to a personality of a person who does not have his/her own wearable device 1. This can improve the safety level and the satisfaction level.

In addition, it is possible to prevent disasters and accidents by sharing health conditions and positional information of members during mountaineering.

In addition, it is possible to clarify individual skiing or snowboarding levels, simplify comparison with skills of other people, and improve skills.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

For example, it is also possible to create a computer program for causing hardware to execute the functions of the wearable device 1, the information processing terminal 2, the communication server 4, the housing case 5, and the wearable device 6. Examples of the hardware include CPUs, ROM, RAM, and the like that are embedded in the wearable device 1, the information processing terminal 2, the communication server 4, the housing case 5, and the wearable device 6. Moreover, it may be possible to provide a computer-readable storage medium having the computer program stored therein.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.

(1) An information processing device including
a generation unit configured to generate behavior recommendation information to a user in a specific environment on a basis of log information related to the user in a daily environment.
(2) The information processing device according to (1),
in which the specific environment is an environment of a sport played above a ground or under water.
(3) The information processing device according to (2),
in which the generation unit generates the behavior recommendation information on a basis of health information that is the log information.
(4) The information processing device according to (3),
in which the generation unit generates the behavior recommendation information further in view of profile information of the user.
(5) The information processing device according to (3) or (4),
in which the generation unit generates the behavior recommendation information further in view of weather.
(6) The information processing device according to any one of (2) to (5),
in which the generation unit generates a dive plan for a dive environment that is the specific environment.
(7) The information processing device according to (6),
in which the generation unit calculates a maximum adequate depth as the dive plan on a basis of a blood pressure value and a maximum diveable depth that are the log information.
(8) The information processing device according to (7),
in which, in a case where age of the user exceeds a threshold, the generation unit modifies the calculated maximum adequate depth.
(9) The information processing device according to any one of (7) and (8), further including
an output unit configured to output the dive plan.
(10) The information processing device according to (6),
in which, in a case where the user is at a beginner diver level, the generation unit calculates a maximum adequate depth and dive duration as the dive plan on a basis of a dive history.
(11) The information processing device according to (10),
in which, in a case where it is recognized that the user is accompanied by a veteran diver on a basis of accompanying member information of the user, the information processing device corrects the maximum adequate depth and the dive duration.
(12) The information processing device according to any one of (6) to (11),
in which the information processing device compares the dive plan with actual dive content of the user, and calculates a score.
(13) The information processing device according to any one of (1) to (12),
in which the generation unit generates the behavior recommendation information further in view of log information or profile information of the user and another accompanying user.
(14) An information processing method including
generating, by a processor, behavior recommendation information to a user in a specific environment on a basis of log information related to the user in a daily environment.
(15) A program causing a computer to function as
a generation unit configured to generate behavior recommendation information to a user in a specific environment on a basis of log information related to the user in a daily environment.

(16) An information processing terminal to be worn by a user, the information processing terminal including a control unit configured to

display behavior recommendation information to the user in a specific environment, the behavior recommendation information being generated on a basis of log information related to the user in a daily environment, and

perform control such that a dive plan that is the behavior recommendation information is compared with a current dive situation of the user, and a warning is output in a case where the current dive situation deviates from the plan.

Reference Signs List

| | |
|---|---|
| 1 | wearable device |
| 10 | control unit |
| 11 | communication unit |
| 12 | operation input unit |
| 13 | sensor |
| 14 | output unit |
| 15 | log information storage unit |
| 16 | time measurement unit |
| 2 | information processing terminal |
| 20 | control unit |
| 201 | log storage control unit |
| 202 | application decision unit |
| 203 | plan generation unit |
| 204 | score calculation unit |
| 21 | communication unit |
| 22 | operation input unit |
| 23 | display unit |
| 24 | personal information DB |
| 25 | personal log information DB |
| 26 | algorithm DB |

## Claims

1. An information processing device comprising a generation unit configured to generate behavior recommendation information to a user in a specific environment on a basis of log information related to the user in a daily environment.

2. The information processing device according to claim 1, wherein the specific environment is an environment of a sport played above a ground or under water.

3. The information processing device according to claim 2, wherein the generation unit generates the behavior recommendation information on a basis of health information that is the log information.

4. The information processing device according to claim 3, wherein the generation unit generates the behavior recommendation information further in view of profile information of the user.

5. The information processing device according to claim 3, wherein the generation unit generates the behavior recommendation information further in view of weather.

6. The information processing device according to claim 2, wherein the generation unit generates a dive plan for a dive environment that is the specific environment.

**7.** The information processing device according to claim 6,
wherein the generation unit calculates a maximum adequate depth as the dive plan on a basis of a blood pressure value and a maximum diveable depth that are the log information.

**8.** The information processing device according to claim 7,
wherein, in a case where age of the user exceeds a threshold, the generation unit modifies the calculated maximum adequate depth.

**9.** The information processing device according to claim 7, further comprising an output unit configured to output the dive plan.

**10.** The information processing device according to claim 6,
wherein, in a case where the user is at a beginner diver level, the generation unit calculates a maximum adequate depth and dive duration as the dive plan on a basis of a dive history.

**11.** The information processing device according to claim 10,
wherein, in a case where it is recognized that the user is accompanied by a veteran diver on a basis of accompanying member information of the user, the information processing device corrects the maximum adequate depth and the dive duration.

**12.** The information processing device according to claim 6,
wherein the information processing device compares the dive plan with actual dive content of the user, and calculates a score.

**13.** The information processing device according to claim 1,
wherein the generation unit generates the behavior recommendation information further in view of log information or profile information of the user and another accompanying user.

**14.** An information processing method comprising
generating, by a processor, behavior recommendation information to a user in a specific environment on a basis of log information related to the user in a daily environment.

**15.** A program causing a computer to function as
a generation unit configured to generate behavior recommendation information to a user in a specific environment on a basis of log information related to the user in a daily environment.

**16.** An information processing terminal to be worn by a user, the information processing terminal comprising
a control unit configured to

display behavior recommendation information to the user in a specific environment, the behavior recommendation information being generated on a basis of log information related to the user in a daily environment, and
perform control such that a dive plan that is the behavior recommendation information is compared with a current dive situation of the user, and a warning is output in a case where the current dive situation deviates from the plan.

FIG. 1

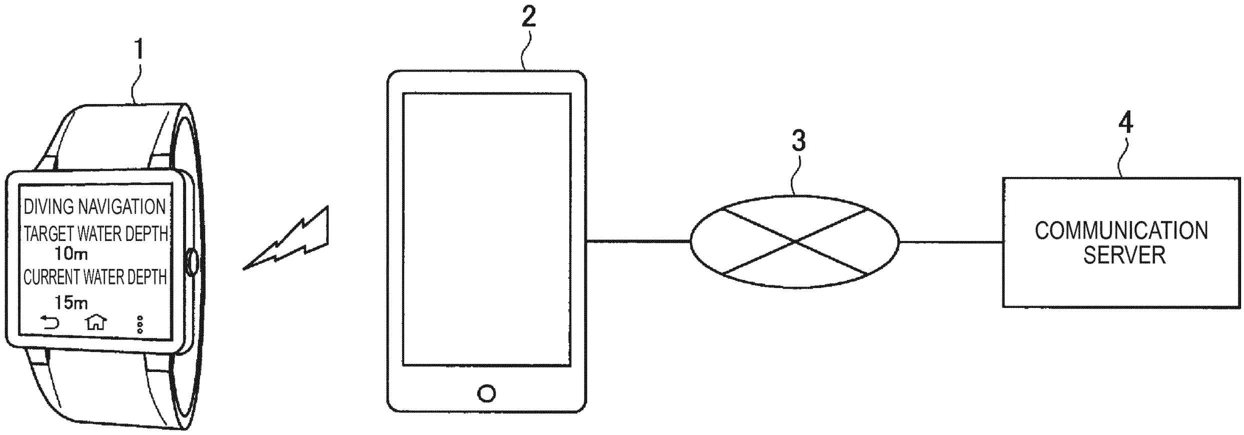
1
DIVING NAVIGATION
TARGET WATER DEPTH
10m
CURRENT WATER DEPTH
15m
2
3
4
COMMUNICATION SERVER

FIG. 2

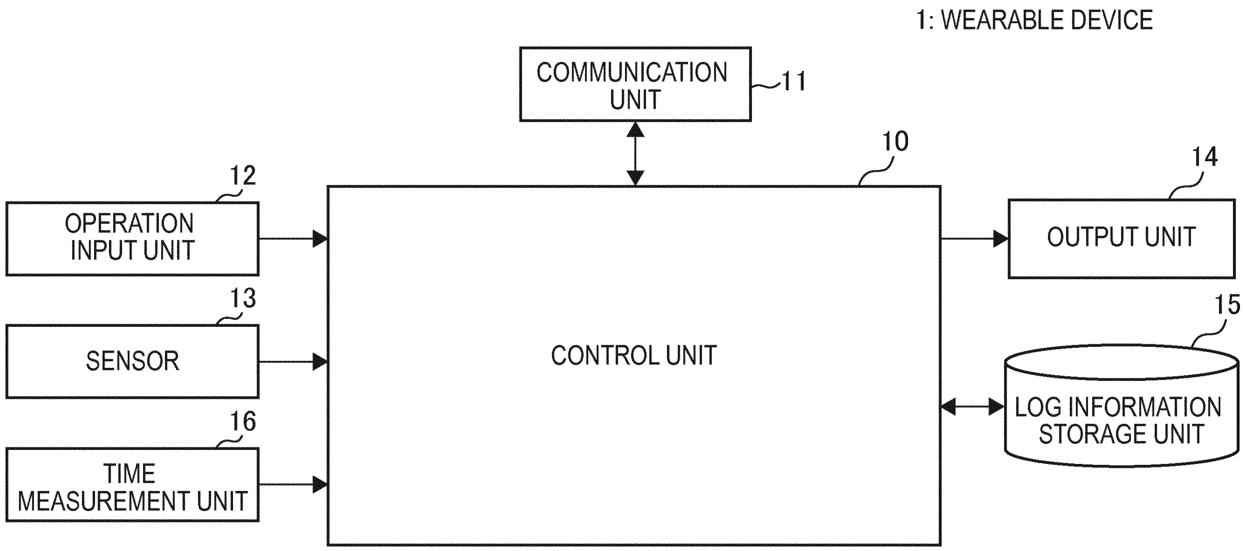
1: WEARABLE DEVICE
COMMUNICATION UNIT
11
10
12
OPERATION INPUT UNIT
13
SENSOR
16
TIME MEASUREMENT UNIT
CONTROL UNIT
14
OUTPUT UNIT
15
LOG INFORMATION STORAGE UNIT

FIG. 3

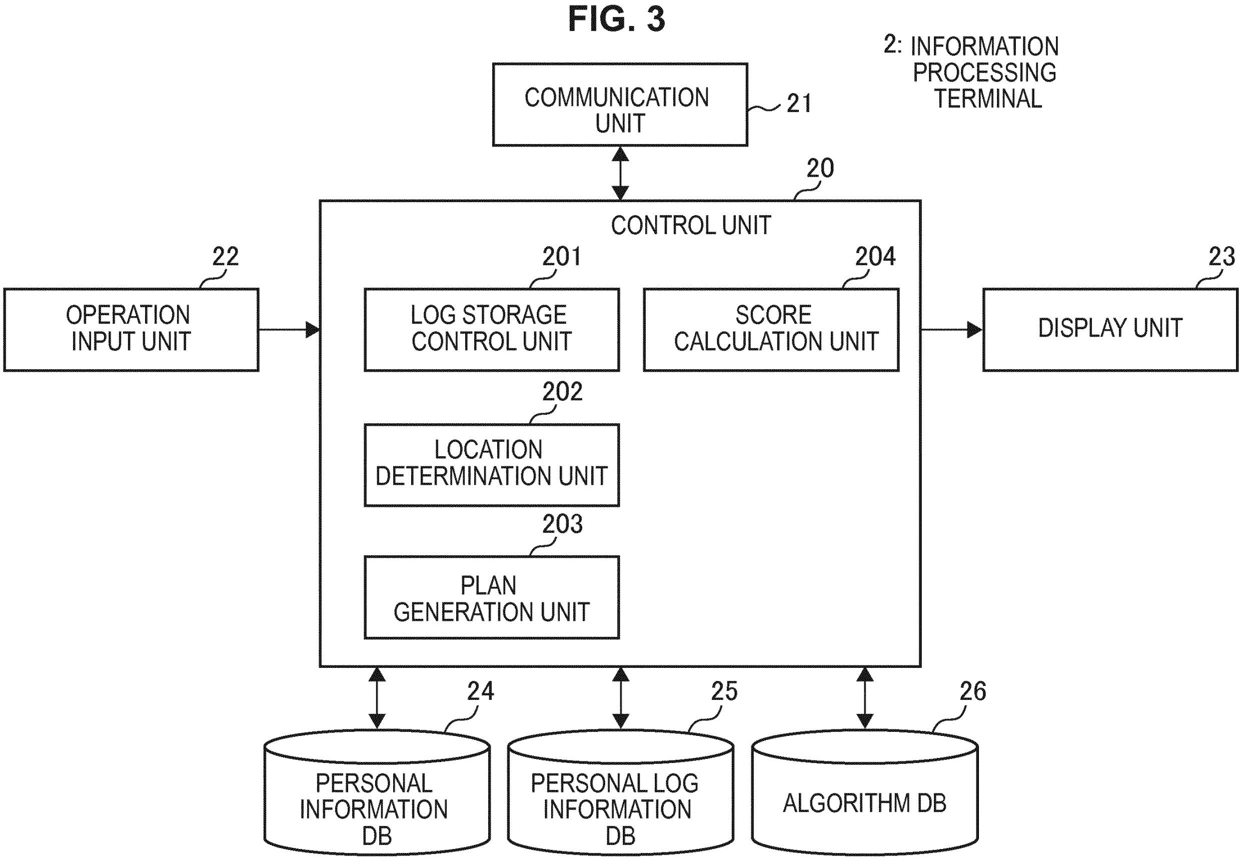
2: INFORMATION PROCESSING TERMINAL
COMMUNICATION UNIT
21
20
CONTROL UNIT
22
OPERATION INPUT UNIT
201
LOG STORAGE CONTROL UNIT
204
SCORE CALCULATION UNIT
23
DISPLAY UNIT
202
LOCATION DETERMINATION UNIT
203
PLAN GENERATION UNIT
24
PERSONAL INFORMATION DB
25
PERSONAL LOG INFORMATION DB
26
ALGORITHM DB

FIG. 4

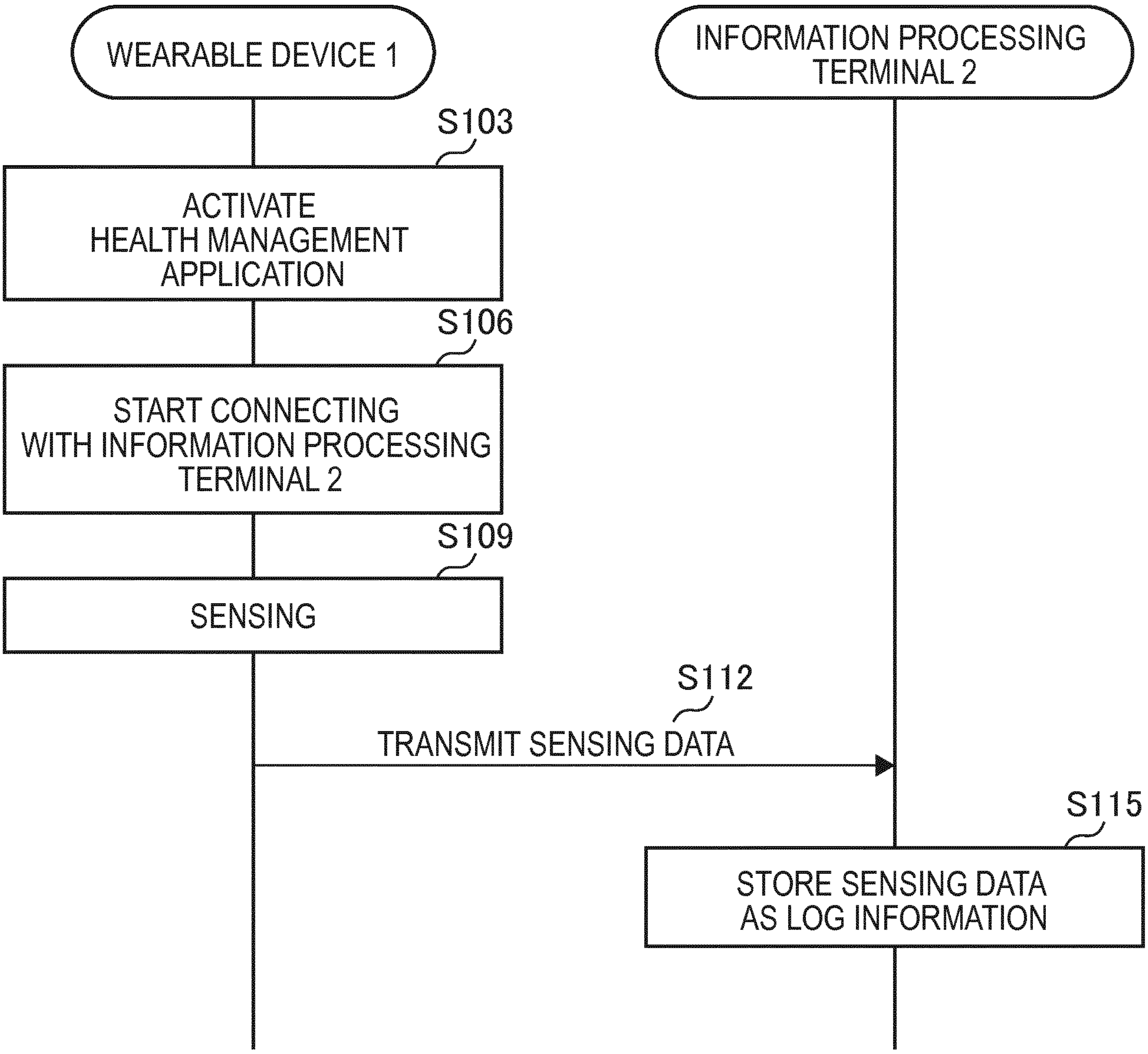
WEARABLE DEVICE 1
INFORMATION PROCESSING TERMINAL 2
S103
ACTIVATE HEALTH MANAGEMENT APPLICATION
S106
START CONNECTING WITH INFORMATION PROCESSING TERMINAL 2
S109
SENSING
S112
TRANSMIT SENSING DATA
S115
STORE SENSING DATA AS LOG INFORMATION

FIG. 5

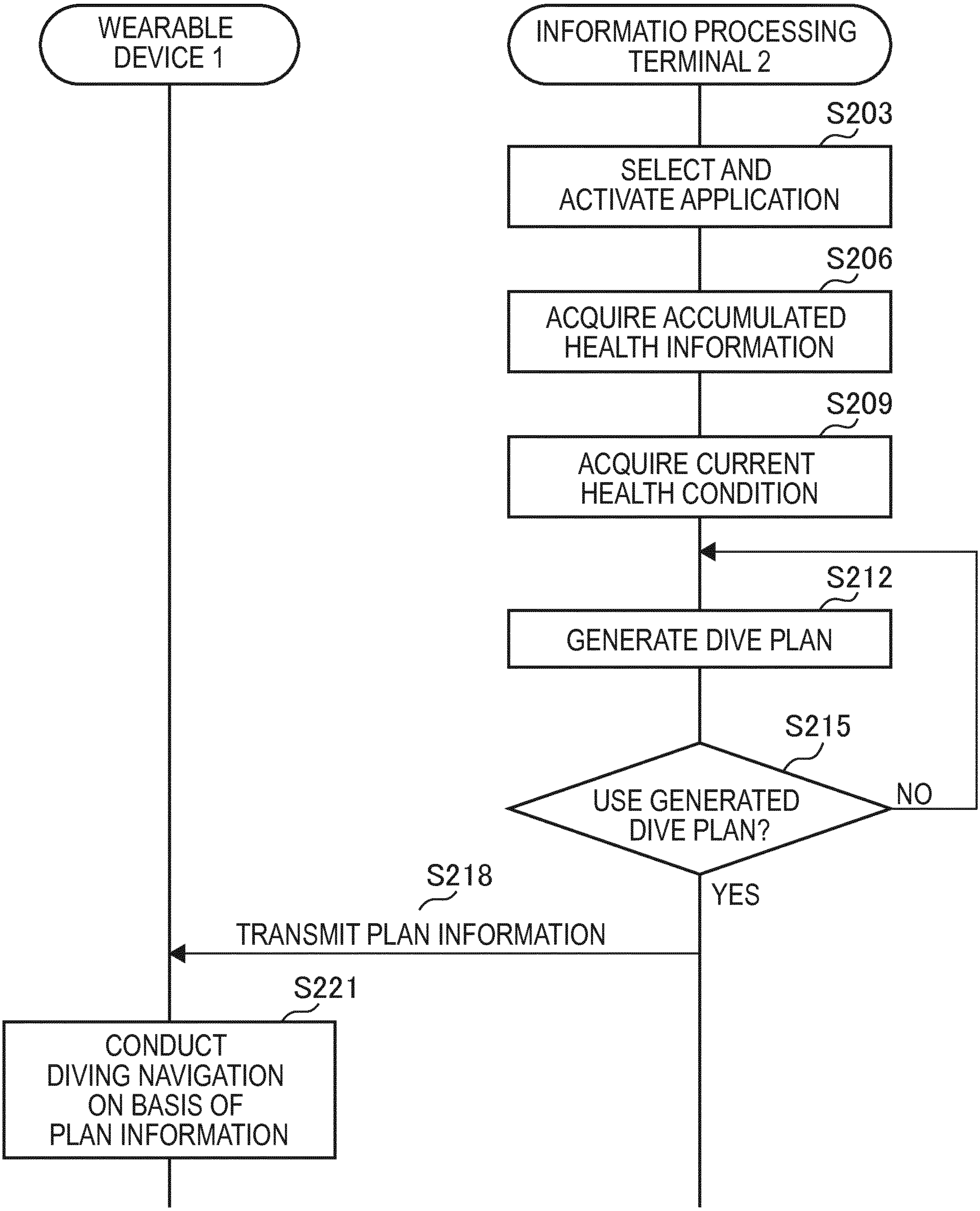
WEARABLE DEVICE 1
INFORMATIO PROCESSING TERMINAL 2
S203
SELECT AND ACTIVATE APPLICATION
S206
ACQUIRE ACCUMULATED HEALTH INFORMATION
S209
ACQUIRE CURRENT HEALTH CONDITION
S212
GENERATE DIVE PLAN
S215
USE GENERATED DIVE PLAN?
NO
YES
S218
TRANSMIT PLAN INFORMATION
S221
CONDUCT DIVING NAVIGATION ON BASIS OF PLAN INFORMATION

FIG. 6

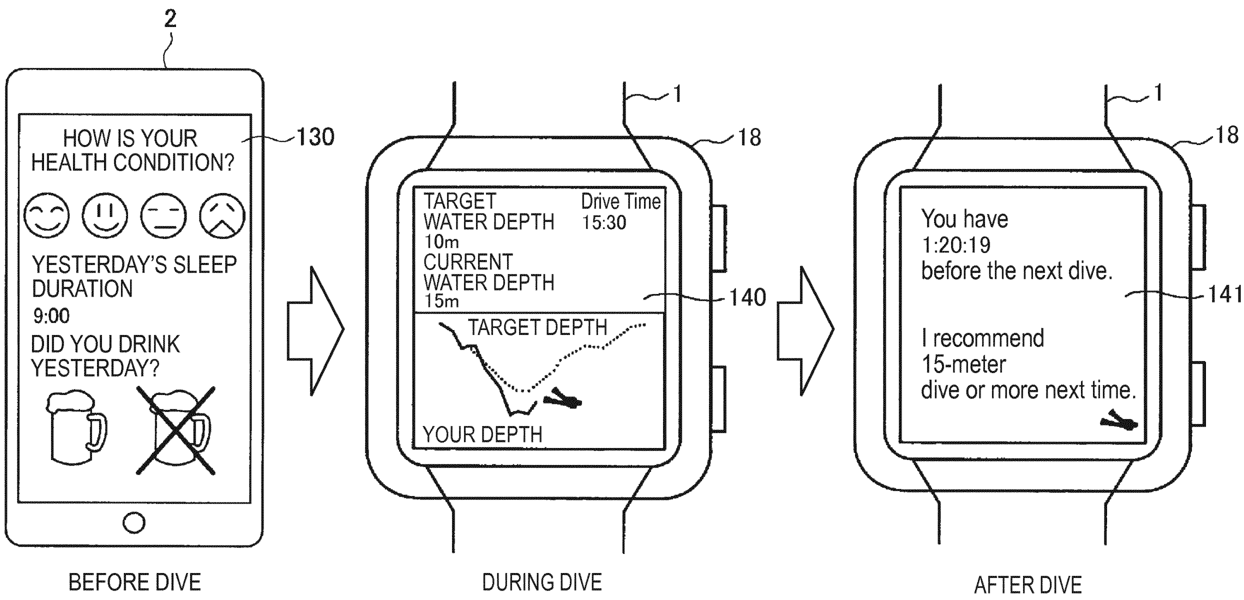
2
HOW IS YOUR HEALTH CONDITION?
YESTERDAY'S SLEEP DURATION
9:00
DID YOU DRINK YESTERDAY?
130
1
18
TARGET WATER DEPTH
10m
Drive Time
15:30
CURRENT WATER DEPTH
15m
140
TARGET DEPTH
YOUR DEPTH
1
18
You have
1:20:19
before the next dive.
141
I recommend
15-meter
dive or more next time.
BEFORE DIVE
DURING DIVE
AFTER DIVE

FIG. 7

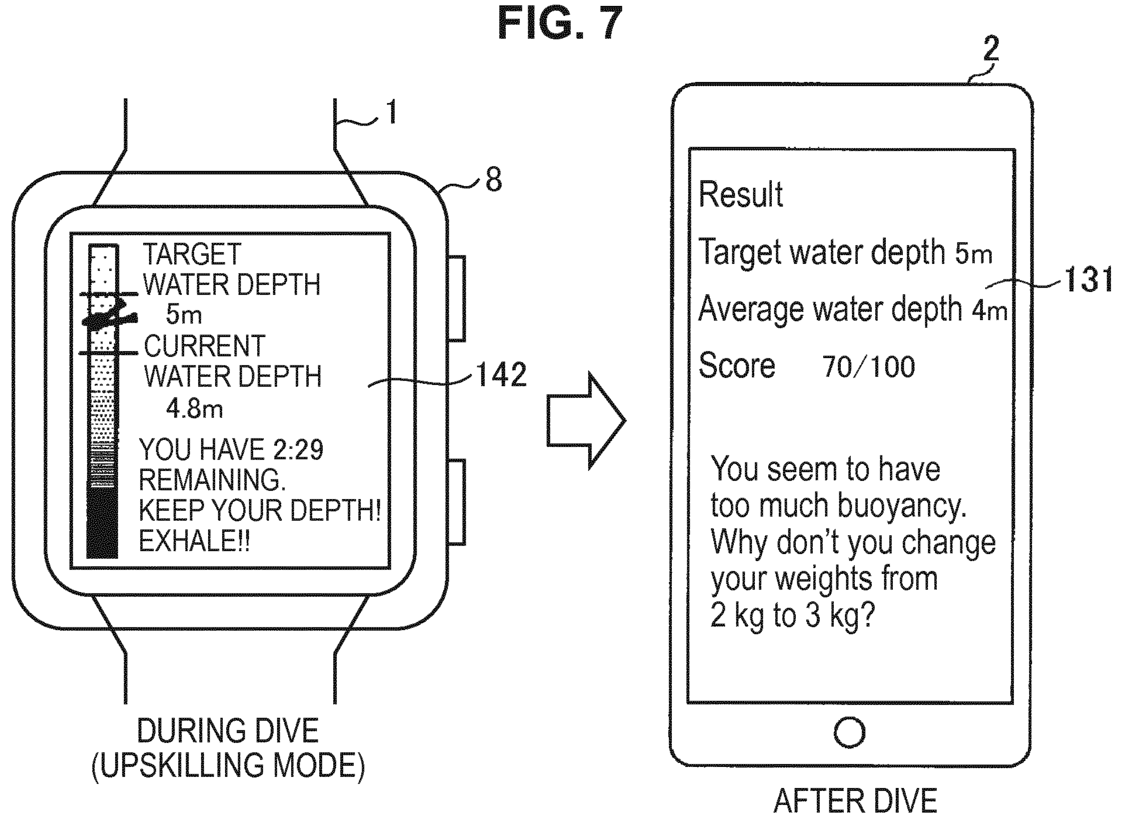
1
8
TARGET
WATER DEPTH
5m
CURRENT
WATER DEPTH
4.8m
YOU HAVE 2:29
REMAINING.
KEEP YOUR DEPTH!
EXHALE!!
142
DURING DIVE
(UPSKILLING MODE)
2
Result
Target water depth 5m
Average water depth 4m
Score 70/100
You seem to have
too much buoyancy.
Why don't you change
your weights from
2 kg to 3 kg?
131
AFTER DIVE

FIG. 8

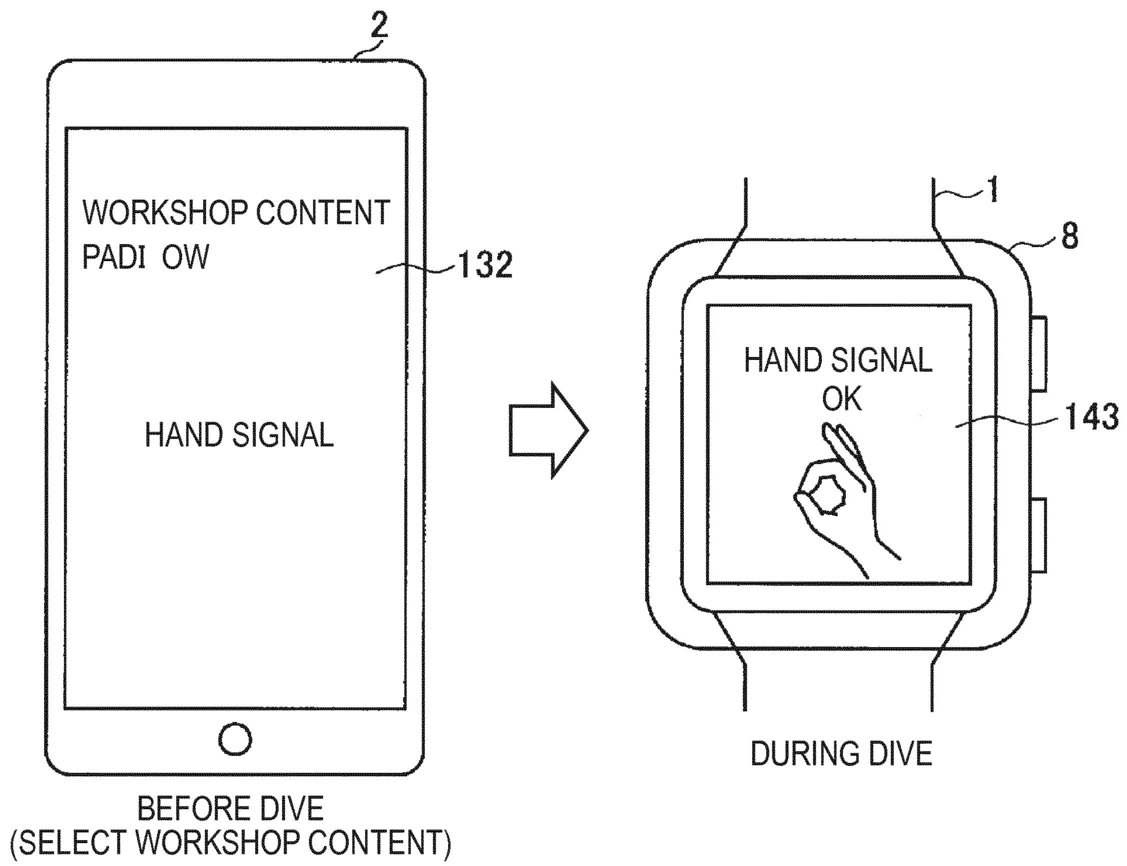
2
WORKSHOP CONTENT
PADI OW
HAND SIGNAL
132
BEFORE DIVE
(SELECT WORKSHOP CONTENT)
1
8
HAND SIGNAL
OK
143
DURING DIVE

FIG. 9

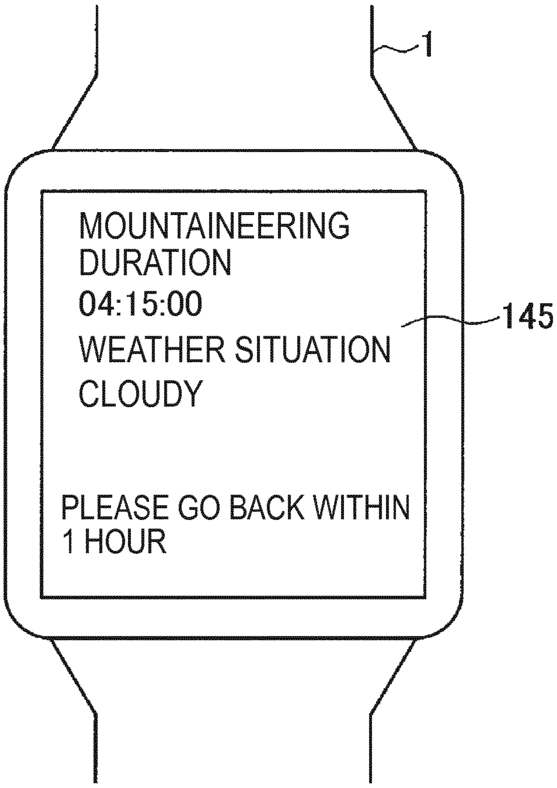
1
MOUNTAINEERING DURATION
04:15:00
WEATHER SITUATION
CLOUDY
PLEASE GO BACK WITHIN 1 HOUR
145

FIG. 10

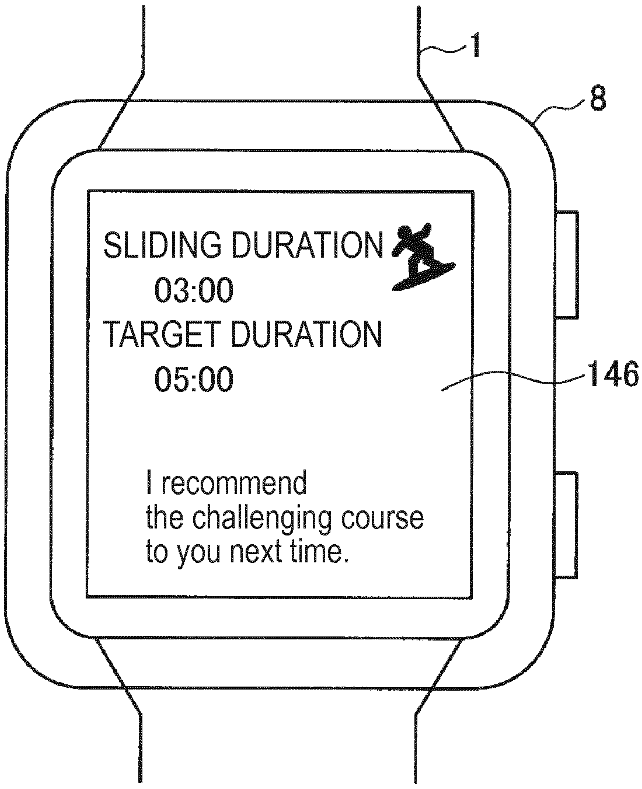
1
8
SLIDING DURATION
03:00
TARGET DURATION
05:00
146
I recommend the challenging course to you next time.

# FIG. 11

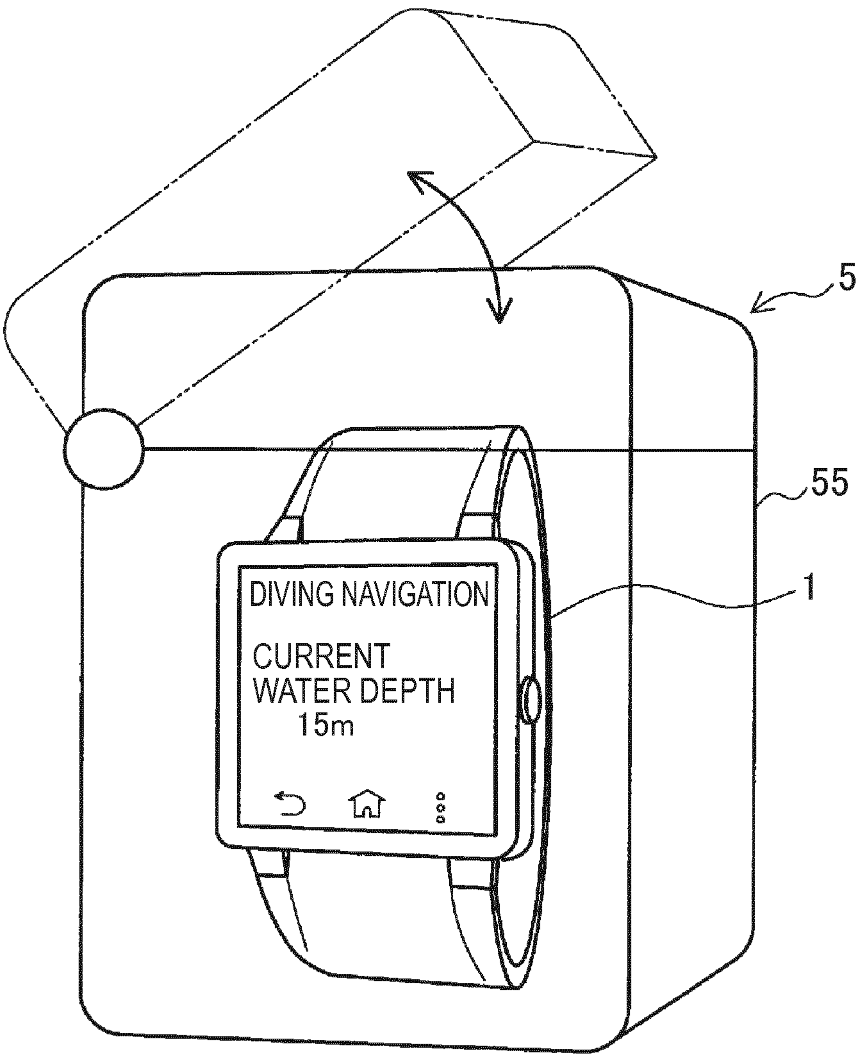

FIG. 12

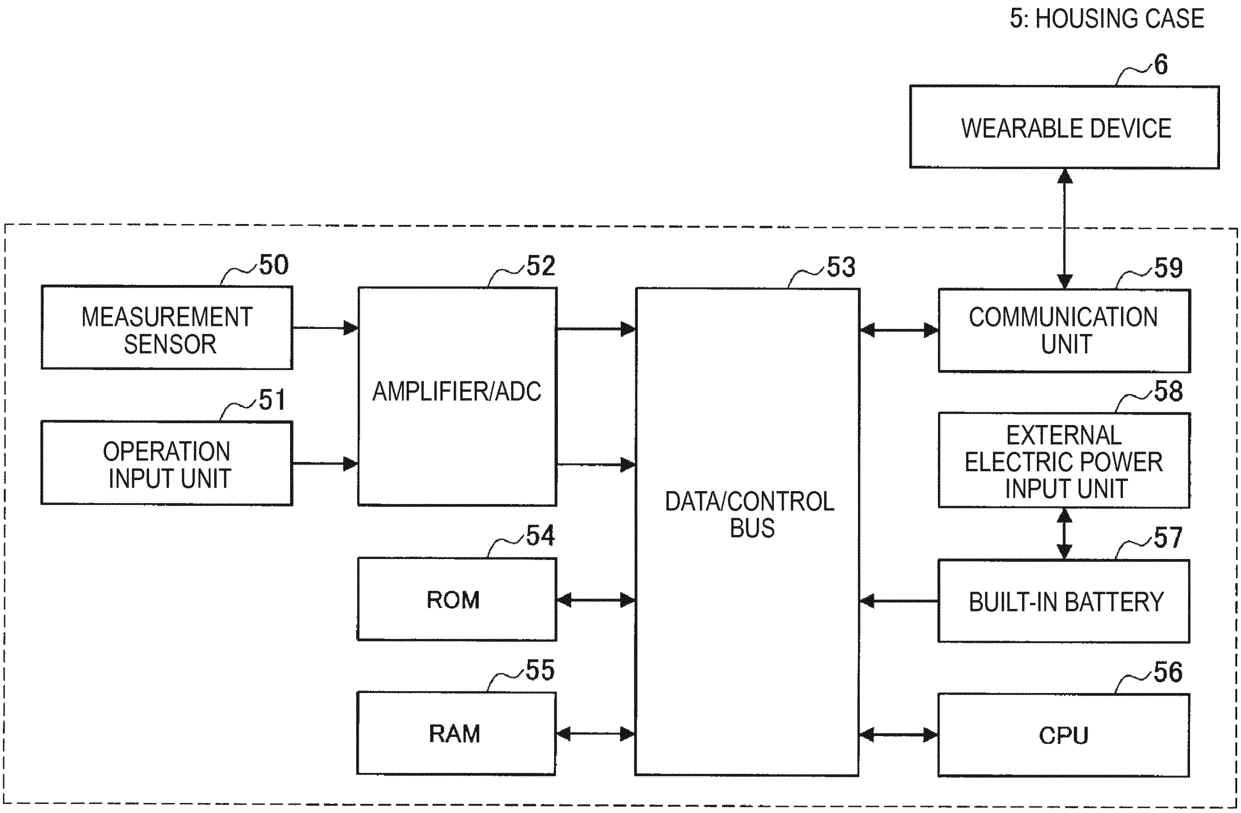
5: HOUSING CASE
6
WEARABLE DEVICE
50
MEASUREMENT SENSOR
51
OPERATION INPUT UNIT
52
AMPLIFIER/ADC
53
DATA/CONTROL BUS
54
ROM
55
RAM
59
COMMUNICATION UNIT
58
EXTERNAL ELECTRIC POWER INPUT UNIT
57
BUILT-IN BATTERY
56
CPU

FIG. 13

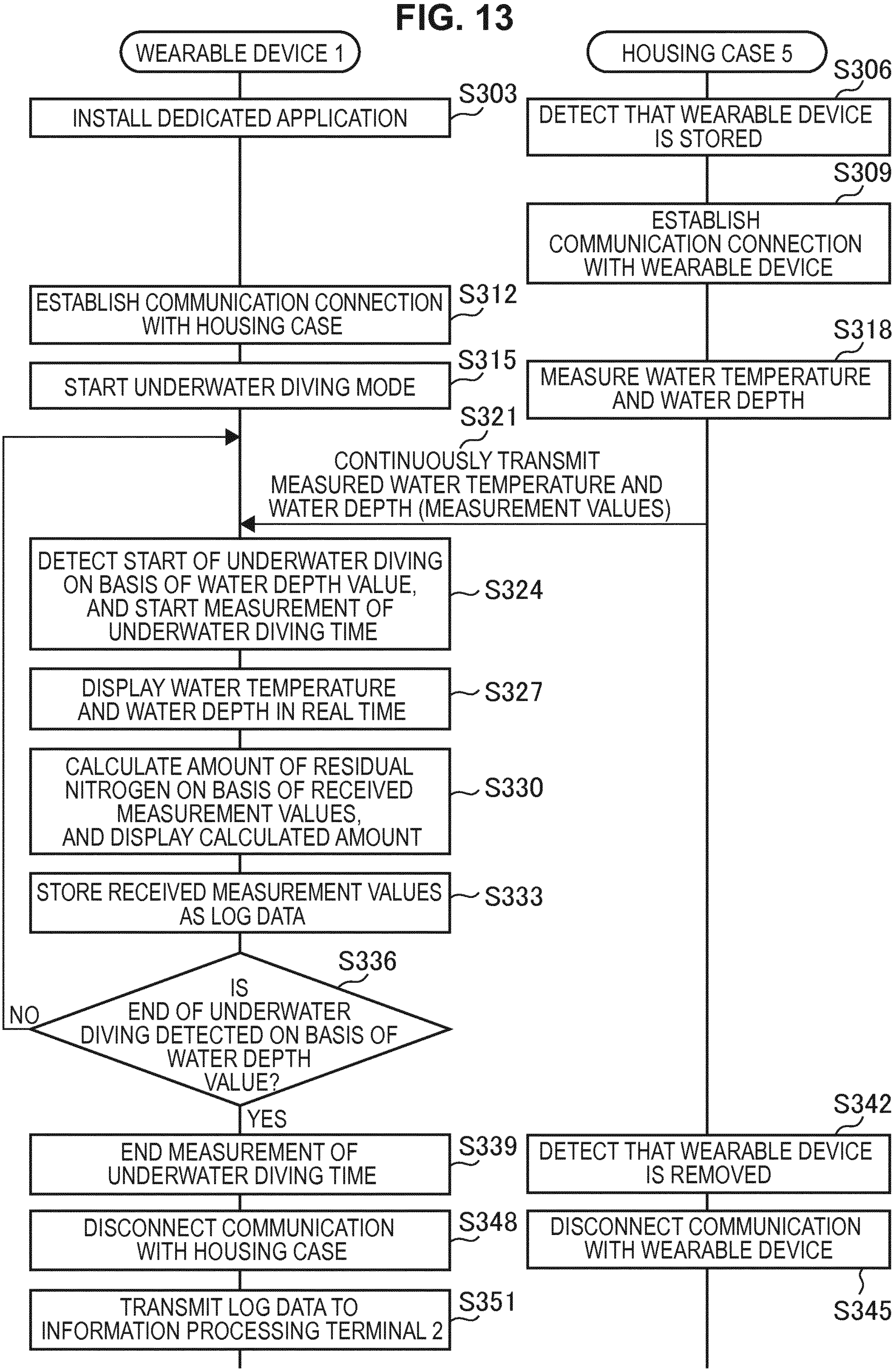
WEARABLE DEVICE 1
HOUSING CASE 5
S303
INSTALL DEDICATED APPLICATION
S306
DETECT THAT WEARABLE DEVICE IS STORED
S309
ESTABLISH COMMUNICATION CONNECTION WITH WEARABLE DEVICE
S312
ESTABLISH COMMUNICATION CONNECTION WITH HOUSING CASE
S315
START UNDERWATER DIVING MODE
S318
MEASURE WATER TEMPERATURE AND WATER DEPTH
S321
CONTINUOUSLY TRANSMIT MEASURED WATER TEMPERATURE AND WATER DEPTH (MEASUREMENT VALUES)
DETECT START OF UNDERWATER DIVING ON BASIS OF WATER DEPTH VALUE, AND START MEASUREMENT OF UNDERWATER DIVING TIME
S324
DISPLAY WATER TEMPERATURE AND WATER DEPTH IN REAL TIME
S327
CALCULATE AMOUNT OF RESIDUAL NITROGEN ON BASIS OF RECEIVED MEASUREMENT VALUES, AND DISPLAY CALCULATED AMOUNT
S330
STORE RECEIVED MEASUREMENT VALUES AS LOG DATA
S333
S336
IS END OF UNDERWATER DIVING DETECTED ON BASIS OF WATER DEPTH VALUE?
NO
YES
END MEASUREMENT OF UNDERWATER DIVING TIME
S339
S342
DETECT THAT WEARABLE DEVICE IS REMOVED
DISCONNECT COMMUNICATION WITH HOUSING CASE
S348
DISCONNECT COMMUNICATION WITH WEARABLE DEVICE
S345
TRANSMIT LOG DATA TO INFORMATION PROCESSING TERMINAL 2
S351

## INTERNATIONAL SEARCH REPORT

International application No.
PCT/JP2016/081374

A. CLASSIFICATION OF SUBJECT MATTER
*B63C11/02*(2006.01)i, *A63B29/00*(2006.01)i, *A63B71/06*(2006.01)i, *A63C11/00*(2006.01)i

According to International Patent Classification (IPC) or to both national classification and IPC

B. FIELDS SEARCHED

Minimum documentation searched (classification system followed by classification symbols)
B63C11/02, A63B29/00, A63B71/06, A63C11/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2013-208266 A (Sony Corp.), 10 October 2013 (10.10.2013), paragraphs [0127] to [0225]; fig. 1 to 14 & US 2013/0261775 A1 paragraphs [0153] to [0260]; fig. 1 to 14 | 1-5,14-15 |
| X<br>Y | JP 2006-021566 A (Seiko Epson Corp.), 26 January 2006 (26.01.2006), paragraphs [0016] to [0048]; fig. 1 to 7 (Family: none) | 1-3,14-16<br>4-13 |
| Y | JP 10-338193 A (Seiko Epson Corp.), 22 December 1998 (22.12.1998), paragraphs [0019] to [0099]; fig. 1 to 9 (Family: none) | 4-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 November 2016 (25.11.16) | 13 December 2016 (13.12.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2016/081374

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-276675 A (Seiko Epson Corp.), 07 October 2004 (07.10.2004), paragraphs [0011] to [0028]; fig. 1 to 4 (Family: none) | 10-13 |
| Y | JP 11-105787 A (Toshiba Corp.), 20 April 1999 (20.04.1999), paragraphs [0007] to [0013] (Family: none) | 13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H8266685 A